# EUROPÄISCHE PATENTSCHRIFT

(11) **EP 4 282 347 B1**
(45) Veröffentlichungstag und Bekanntmachung des Hinweises auf die Patenterteilung: **12.03.2025**
(21) Anmeldenummer: 23174532.4
(22) Anmeldetag: 22.05.2023
(51) Int. Cl.: A61B 17/15

(54) **CHIRURGISCHES INSTRUMENTENSYSTEM**
SURGICAL INSTRUMENT SYSTEM
SYSTÈME D'INSTRUMENT CHIRURGICAL

(30) Priorität: 24.05.2022 DE 102022205193
(43) Veröffentlichungstag der Anmeldung: 29.11.2023
(73) Patentinhaber: Aesculap AG, 78532 Tuttlingen (DE)
(72) Erfinder: Firmbach, Franz-Peter, 78576 Emmingen-Liptingen (DE); Anhorn, Svenja, 72535 Heroldstatt (DE)
(74) Vertreter: Patentanwälte Ruff, Wilhelm, Beier, Dauster & Partner mbB

(56) Entgegenhaltungen:
- WO-A1-97/21390
- DE-A1- 102015 100 049
- DE-T2- 69 636 636
- DE-T2- 69 722 052
- US-A1- 2016 030 053

## Beschreibung

Die Erfindung betrifft ein chirurgisches Instrumentensystem nach dem Oberbegriff des Anspruchs 1.

Die Verwendung orthopädischer Prothesen als künstlicher Ersatz für beschädigte oder abgenutzte natürlichen Knochenstrukturen eines Patienten ist gängige medizinische Praxis. Insbesondere Hüft- und Kniegelenkersatzoperationen gehören mittlerweile zum Standardrepertoire der chirurgischen Orthopädie.

Bei einer Kniegelenkersatzoperation (englisch: Total Knee Arthroplasty (TKA)) werden abgenutzte oder anderweitig durch Krankheit oder einen Unfall beeinträchtigte Gelenkflächen des Femurs und/oder der Tibia durch eine Kniegelenkprothese ersetzt. Solche Kniegelenkprothesen umfassen üblicherweise eine Femurkomponente, welche am distalen Ende des Femurs implantiert wird, und eine Tibiakomponente, welche am proximalen Ende der Tibia implantiert wird. Um eine einwandfreie Funktion des künstlichen Gelenkersatzes zu gewährleisten, müssen die besagten Komponenten hinsichtlich ihrer Lage und Orientierung in Bezug auf die Anatomie des Patienten und dessen Körperachsen in definierter Weise möglichst präzise positioniert werden. Andernfalls ist mit einem für den Patienten nicht zufriedenstellenden Ergebnis zu rechnen. Hinsichtlich der Positionierung der Komponenten existieren unterschiedliche chirurgische Ansätze.

Ein als Mechanical Alignment bekannter und bislang üblicher Ansatz sieht vor, dass die Position und Ausrichtung der künstlichen Gelenkachsen der Kniegelenkprothese mechanisch ideal und insoweit ohne Berücksichtigung etwaiger orthopädischer Fehlstellungen des Patienten ausgerichtet werden. Hierbei dient oftmals die Längsachse der Tibia als Referenzachse für die Ausrichtung und Positionierung. Klinische Studien haben gezeigt, dass der Mechanical Alignment-Ansatz zu einer als unnatürlich empfundenen Funktion des künstlichen Kniegelenks führen kann.

Als ein weiterer Ansatz ist das sog. Kinematic Alignment bekannt. Bei dieser Vorgehensweise werden die Femurkomponente und die Tibiakomponente unter Berücksichtigung etwaiger orthopädischer Fehlstellungen des Patienten positioniert. Ziel hierbei ist es, die natürliche und unter Umständen mit Fehlstellungen behaftete Gelenkausrichtung des Patienten wiederherzustellen. Klinische Studien haben gezeigt, dass der Kinematic Alignment-Ansatz oftmals mit einer verbesserten Patientenzufriedenheit einhergeht. Insbesondere die Funktion des künstlichen Kniegelenks wird seitens der Patienten als eher natürlich empfunden.

Mit dem Bestreben, die Patientenzufriedenheit weiter zu verbessern, geht ein grundsätzlicher Bedarf nach möglichst präzisen, einfach verwendbaren und kosteneffizienten chirurgischen Instrumenten und Instrumentensystemen zur Umsetzung des Kinematic Alignment einher.

Die vorliegende Erfindung befasst sich mit solchen chirurgischen Instrumentensystemen, im Speziellen mit einem chirurgischen Instrumentensystem zur Positionierung eines distalen Femurschnittblocks im Rahmen des Kinematic Alignment.

Ein derartiges chirurgisches Instrumentensystem ist aus der US 10,130,375 B2 bekannt und weist einen intramedullären Stab und einen Referenzblock auf. Der intramedulläre Stab ist zwischen einem proximalen Stabende und einem distalen Stabende längserstreckt und zum Einbringen in den Markraum eines Femurs eingerichtet. Der Referenzblock weist eine proximal orientierte Blockrückseite auf, welche zur Anlage an den distalen Kondylen des Femurs eingerichtet ist. Zudem weist der Referenzblock eine Fixiervorrichtung zur lösbaren Fixierung eines distalen Femurschnittblocks auf. Zur femurseitigen Positionierung ist der Referenzblock ausgehend von dem distalen Stabende in proximaler Richtung auf den intramedullären Stab aufschiebbar. Zu diesem Zweck weist der Referenzblock des bekannten chirurgischen Instrumentensystems ein Langloch auf. Das Langloch ist axial zwischen der Blockrückseite und einer distal gegenüberliegenden Blockvorderseite erstreckt und mediolateral länglich ausgedehnt. Hierdurch ist der Referenzblock in aufgeschobenem Zustand mediolateral begrenzt relativbeweglich. Zudem ist der Referenzblock um eine anteroposterior orientierte Kippachse relativ zu dem intramedullären Stab kippbeweglich, wobei eine Position der Kippachse in Bezug auf den in dem Langloch aufgenommenen intramedullären Stab nicht eindeutig definiert und in festen Grenzen veränderlich ist.

Aus der DE 696 36 636 T2, der DE 697 22 052 T2 und der DE 10 2015 100049 A1 ist jeweils ein chirurgisches Instrumentensystem mit den oberbegrifflichen Merkmalen des Anspruchs 1 bekannt.

Aufgabe der Erfindung ist es, ein chirurgisches Instrumentensystem der eingangs genannten Art bereitzustellen, welches eine präzise Einstellung der Lage und Orientierung des Referenzblocks und letztlich verbesserte Operationsergebnisse ermöglicht.

Diese Aufgabe wird durch das Bereitstellen eines chirurgischen Instrumentensystems mit den Merkmalen des Anspruchs 1 gelöst. Durch die erfindungsgemäße Lösung wird eine verbesserte Lagerung des Referenzblocks an dem intramedullären Stab erreicht. Im Vergleich zu aus dem Stand der Technik bekannten Lösungen ermöglicht die Erfindung eine kinematisch wohldefinierte Relativbeweglichkeit des Referenzblocks. Hierdurch können die Lage und Orientierung des Referenzblocks präzise eingestellt werden. Insbesondere kann durch die Schwenkbeweglichkeit des Referenzblocks um die anteroposterior orientierte Schwenkachse der sogenannte Varus-Valgus-Winkel präzise eingestellt werden. Hierdurch werden Fehlausrichtungen der kinematischen Achsen des Kniegelenkersatzes vermieden und letztlich verbesserte Operationsergebnisse ermöglicht. Der Referenzblock ist mittelbar, nämlich mittels des Lagerelements, an dem intramedullären Stab gelagert oder lagerbar. Zu diesem Zweck weist das Lagerelement die besagte Aufnahmebohrung auf. Die Aufnahmebohrung ist maßlich komplementär zu dem intramedullären Stab gestaltet. In aufgeschobenem Zustand des Referenzblocks sind die Aufnahmebohrung und der intramedulläre Stab koaxial zueinander ausgerichtet und der intramedulläre Stab ist radial formschlüssig in der Aufnahmebohrung aufgenommen. Infolge des Formschlusses sind das Lagerelement und der intramedulläre Stab mediolateral sowie anteroposterior relativ zueinander festgelegt. Die besagte Schwenkbeweglichkeit des Referenzblocks in Relation zu dem intramedullären Stab wird durch die schwenkbewegliche Lagerung des Lagerelements an dem Referenzblock ermöglicht. Die Lagerung kann beispielsweise als Gleitführung, Achsführung oder dergleichen ausgebildet sein und Bauteile und/oder Abschnitte umfassen, die zum einen an dem Referenzblock und zum anderen an dem Lagerelement angeordnet und/oder ausgebildet sind.

Die in dieser Beschreibung verwendeten Lage- und Richtungsbezeichnungen beziehen sich auf den Körper eines Patienten, insbesondere dessen Femur, und sind insoweit gemäß ihrer üblichen anatomischen Bedeutung zu verstehen. Folglich bedeutet "anterior" vordere(r) oder vorne liegend, "posterior" hintere(r) oder hinten liegend, "medial" innere(r) oder innen liegend, "lateral" äußere(r) oder außen liegend, "proximal" zum Körperzentrum hin und "distal" vom Körperzentrum entfernt. Weiter bedeuten "proximodistal" entlang, vorzugsweise parallel zu, einer proximal-distal ausgerichteten Achse, "anteroposterior" entlang, vorzugsweise parallel zu, einer anterior-posterior ausgerichteten Achse und "mediolateral" entlang, vorzugsweise parallel zu, einer medial-lateral ausgerichteten Achse. Die besagten Achsen sind orthogonal zueinander ausgerichtet und können selbstverständlich in Bezug zu nicht mit der Anatomie des Patienten in Verbindung stehenden X-, Y- und Z-Achsen gesetzt werden. Beispielsweise kann die proximaldistale Achse alternativ als X-Achse bezeichnet werden. Die medial-laterale Achse kann als Y-Achse bezeichnet werden. Die anterior-posteriore Achse kann als Z-Achse bezeichnet werden. Zur verbesserten Veranschaulichung und der Einfachheit der Bezeichnung wegen werden nachfolgend in erster Linie die besagten anatomischen Lage- und Richtungsbezeichnungen verwendet. Weiter werden Bezeichnungen wie "Rückseite" einer Komponente oder eines Abschnitts des chirurgischen Instrumentensystems, beispielsweise des Referenzblocks, in Bezug auf eine proximal gerichtete Blickrichtung verwendet. Dementgegen werden Bezeichnungen wie "Vorderseite" in Bezug auf eine distal gerichtete Blickrichtung verwendet.

In Ausgestaltung der Erfindung weist der Referenzblock eine Gleitführung mit mediolateral gegenüberliegenden kreisbogenförmig konkav gekrümmten Führungsflächen auf, und das Lagerelement weist mediolateral gegenüberliegende kreisbogenförmig konvex gekrümmte Gleitflächen auf, wobei die Gleitflächen und die Schwenkachse rotatorisch gleitbeweglich und proximodistal sowie mediolateral translatorisch formschlüssig mit den Führungsflächen zusammenwirken. Hierdurch wird eine konstruktiv besonders einfache und dennoch präzise und robuste schwenkbewegliche Lagerung zwischen dem Lagerelement und dem Referenzblock erreicht. Die Führungsflächen und die Gleitflächen sind jeweils in Bezug auf eine anterior oder posterior gerichtete Blickrichtung kreisbogenförmig. Die Führungsflächen und die Gleitflächen sind zueinander komplementär gekrümmt, wobei die Führungsflächen konkav und die Gleitflächen jeweils konvex sind. Die Führungsflächen definieren einen Innendurchmesser der Gleitführung. Die Gleitflächen definieren einen komplementären Außendurchmesser. Der Außendurchmesser ist in den Innendurchmesser eingepasst. Die Passung ist vorzugsweise derart gewählt, dass eine präzise, spielfreie und gleichzeitig ausreichend leichtgängige Relativbeweglichkeit zwischen dem Lagerelement und dem Referenzblock erreicht ist. Vorzugsweise ist die Aufnahmebohrung in mediolateraler Richtung, bevorzugt mittig, zwischen den gegenüberliegenden Gleitflächen des Lagerelements angeordnet. Die gegenüberliegenden Führungsflächen können auch als erste Führungsfläche und zweite Führungsfläche oder alternativ als mediale Führungsfläche und laterale Führungsfläche bezeichnet werden. Entsprechendes gilt mutatis mutandis hinsichtlich der Gleitflächen.

In weiterer Ausgestaltung der Erfindung sind die Führungsflächen jeweils parallel zu der Schwenkachse gerade längserstreckt, wobei die Gleitflächen entlang der Schwenkachse translatorisch beweglich mit den Führungsflächen zusammenwirken. Hierdurch ist der Referenzblock anteroposterior relativ zu dem intramedullären Stab gleitbeweglich. Dies erlaubt eine Einstellung der anteroposterioren Position des Referenzblocks. Hierdurch ist die Positionierung des Referenzblocks in verbesserter Weise an eine gegebene Größe des Femurs anpassbar. Infolge ihrer geraden Längserstreckung bilden die gegenüberliegenden Führungsflächen gleichsam jeweils eine Führungsbahn. Der Referenzblock ist entlang der Schwenkachse und/oder der Führungsbahn zwischen einer oberen Endlage und einer unteren Endlage relativ zu dem Lagerelement translatorisch verlagerbar.

In weiterer Ausgestaltung der Erfindung weist der Referenzblock einen von der Blockrückseite auf eine distal gegenüberliegende Blockvorderseite reichenden sowie parallel zu der Schwenkachse gerade längserstreckten Aufnahmeschlitz mit mediolateral gegenüberliegenden Innenwandungen auf, an welchen die Führungsflächen angeordnet und/oder ausgebildet sind, wobei der Aufnahmeschlitz zum Einführen des Lagerelements eine anterior an einer Blockoberseite oder posterior an einer Blockunterseite des Referenzblocks angeordnete Einführöffnung aufweist. Der Aufnahmeschlitz ermöglicht eine besonders einfache und zeitsparende Anbringung des Lagerelements an dem Referenzblock oder umgekehrt. Beispielsweise kann zunächst das Lagerelement mit der Aufnahmebohrung voran in proximaler Richtung auf das distale Stabende aufgeschoben werden. Hiernach kann der Referenzblock entlang der Schwenkachse anterior oder posterior, je nachdem ob die Einführöffnung auf der Blockober- oder -unterseite angeordnet ist, auf das Lagerelement aufgeschoben werden. Alternativ kann das Lagerelement vor dem Aufschieben auf den intramedullären Stab in den Aufnahmeschlitz eingebracht werden. Bei einer Ausgestaltung ist die Einführöffnung an der Blockoberseite des Referenzblocks angeordnet. Bei einer weiteren Ausgestaltung ist die Einführöffnung auf der Blockunterseite angeordnet. Die Blockoberseite und die Blockunterseite liegen einander anteroposterior gegenüber. Vorzugsweise unterteilt der Aufnahmeschlitz den Referenzblock in einen lateralen Blockabschnitt und einen medialen Blockabschnitt. Eine Rückseite des medialen Blockabschnitts ist zur Anlage an der medialen distalen Kondyle des Femurs eingerichtet. Eine Rückseite des lateralen Blockabschnitts ist zur Anlage an der lateralen distalen Kondyle des Femurs eingerichtet.

In weiterer Ausgestaltung der Erfindung weist der Referenzblock eine Achsführung mit wenigstens einem koaxial zu der Schwenkachse orientierten Achselement auf, und das Lagerelement weist wenigstens eine koaxial zu der Schwenkachse orientierte Achsaufnahme auf, wobei das Achselement um die Schwenkachse gleitbeweglich in die Achsaufnahme eingreift. Das Achselement ist vorzugsweise stift-, bolzen- und/oder zapfenförmig gestaltet, so dass auch von einem Achsstift, Achsbolzen oder Achszapfen gesprochen werden kann. Die Achsführung ist konstruktiv vergleichsweise einfach aufgebaut. Dies erlaubt eine einfache Herstellung und Montage sowie damit einhergehende Kosteneinsparungen. Gleichzeitig kann eine dennoch hinreichend präzise schwenkbewegliche Lagerung erreicht werden. Bei einer Ausgestaltung weist der Referenzblock zwei Achselemente und das Lagerelement dementsprechend zwei Achsaufnahmen auf, die mit jeweils einem der Achselemente zusammenwirken. In diesem Zusammenhang kann auch von einem anterioren Achselement und einem posterioren Achselement gesprochen werden. Entsprechendes gilt mutatis mutandis hinsichtlich der Achsaufnahmen. Bei der besagten Ausgestaltung ist die Aufnahmebohrung vorzugsweise anteroposterior, bevorzugt mittig, zwischen der anterioren Achsaufnahme und der posterioren Achsaufnahme angeordnet.

Weiter gemäß der Erfindung ist das wenigstens eine Lagerelement lösbar an dem Referenzblock gelagert, und es sind mehrere unterschiedliche Lagerelemente mit unterschiedlich geneigt längserstreckten Aufnahmebohrungen vorhanden, welche zur Einstellung eines Flexions-Extensions-Winkels des Referenzblocks an demselben gegeneinander austauschbar lagerbar sind. Die mehreren unterschiedlichen Lagerelemente unterscheiden sich vorzugsweise ausschließlich im Hinblick auf die Neigung der Aufnahmebohrung voneinander. Vorzugsweise sind die unterschiedlichen Lagerelemente im Übrigen identisch. Infolge der unterschiedlich geneigt längserstreckten Aufnahmebohrungen kann der Neigungswinkel des Referenzblocks - durch eine entsprechende Auswahl des jeweiligen Lagerelements - auf einfache und präzise Weise eingestellt werden. Die Aufnahmebohrungen sind unterschiedlich geneigt in Bezug auf die proximal orientierte Blockrückseite des Referenzblocks. Dabei sind die Aufnahmebohrungen relativ zu der Normalenrichtung der Blockrückseite unterschiedlich in anteriorer Richtung und/oder posteriorer Richtung geneigt. Dies erlaubt eine einfache und präzise Einstellung des Flexions-Extensions-Winkels des Referenzblocks, d.h. dessen Rotationswinkel um eine mediolateral ausgerichtete Achse.

In weiterer Ausgestaltung der Erfindung sind mehrere unterschiedliche Kompensationselemente mit unterschiedlichen proximodistalen Dicken vorhanden und zur Kompensation unterschiedlich stark ausgeprägter Abnutzungen der posterioren Kondylen an der Blockrückseite gegeneinander austauschbar anbringbar. Das chirurgische Instrumentensystem ist, wie eingangs erläutert, zur Umsetzung des Kinematic Alignment-Ansatzes vorgesehen. Dieser Ansatz sieht üblicherweise eine maßliche Kompensation der besagten kondylären Abnutzungen vor. Zu diesem Zweck weist das chirurgische Instrumentensystem bei dieser Ausgestaltung der Erfindung mehrere unterschiedliche und gegeneinander austauschbare Kompensationselemente mit unterschiedlicher proximodistaler Dicke auf.

In weiterer Ausgestaltung der Erfindung ist ein Schnittblock vorhanden, welcher zu einer Schnittführung an den distalen Kondylen des Femurs eingerichtet und an einer Fixiervorrichtung des Referenzblocks lösbar fixiert oder fixierbar ist. Der Schnittblock kann auch als Sägeblock oder cutting jig bezeichnet werden. Der Schnittblock weist wenigstens einen Führungsschlitz zur Aufnahme und Führung eines Sägeblatts auf. Die grundsätzliche Gestaltung und Funktionsweise eines solchen Schnittblocks sind dem Fachmann bekannt. Die Fixiervorrichtung ist vorzugsweise im Bereich der anterioren Blockoberseite des Referenzblocks angeordnet.

In weiterer Ausgestaltung der Erfindung weist der intramedulläre Stab eine Länge zwischen 120 mm und 250 mm, bevorzugt zwischen 150 mm und 220 mm, besonders bevorzugt zwischen 170 mm und 200 mm, auf.

Weitere Vorteile und Merkmale der Erfindung ergeben sich aus den Ansprüchen sowie aus der nachfolgenden Beschreibung bevorzugter Ausführungsbeispiele der Erfindung, die anhand der Zeichnungen darrgestellt sind.
- Fig. 1: zeigt in schematischer Perspektivdarstellung eine Ausführungsform eines erfindungsgemäßen chirurgischen Instrumentensystems mit einem intramedullären Stab, einem Referenzblock, einem Lagerelement und einem Schnittblock,
- Fig. 2: in schematischer Perspektivdarstellung den Referenzblock und das Lagerelement des chirurgischen Instrumentensystems nach Fig. 1 in einer auf eine Blockrückseite gerichteten Blickrichtung,
- Fig. 3: in schematischer Perspektivdarstellung eine intraoperative Situation unter Verwendung des chirurgischen Instrumentensystems nach Fig. 1,
- Fig. 4: das chirurgische Instrumentensystem nach Fig. 1 in einer schematischen Seitenansicht mit lateral gerichteter Blickrichtung,
- Fig. 5: eine schematische Längsschnittdarstellung entlang einer Schnittlinie B-B gemäß Fig. 4,
- Fig. 6: das chirurgische Instrumentensystem nach den Fig. 1 bis 5 in einer schematischen Aufsicht mit posterior gerichteter Blickrichtung,
- Fig. 7: eine schematische Längsschnittdarstellung entlang einer Schnittlinie A-A gemäß Fig. 6,
- Fig. 8: in schematischer Perspektivdarstellung unterschiedliche Lagerelemente mit unterschiedlich geneigten Aufnahmebohrungen,
- Fig. 9: in schematischer Perspektivdarstellung eine weitere Ausführungsform eines erfindungsgemäßen chirurgische Instrumentensystems in einer intraoperativen Situation,
- Fig. 10: in schematischer Perspektivdarstellung den Referenzblock und das Lagerelement des chirurgischen Instrumentensystems nach Fig. 9,
- Fig. 11: das chirurgische Instrumentensystem nach Fig. 9 in einer schematischen Aufsicht mit posterior gerichteter Blickrichtung,
- Fig. 12: ein schematischer Längsschnitt entlang einer Schnittlinie A-A gemäß Fig. 11,
- Fig. 13: das chirurgische Instrumentensystem nach den Fig. 9 bis 12 in einer schematischen Seitenansicht mit medial gerichteter Blickrichtung und
- Fig. 14: eine schematische Längsschnittdarstellung entlang einer Schnittlinie C-C gemäß Fig. 13.

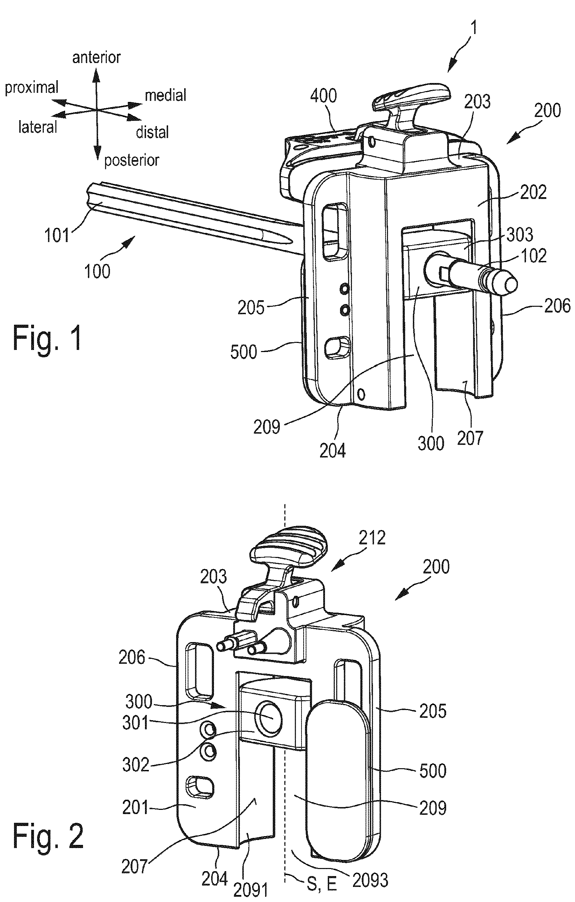

Gemäß den Fig. 1 bis 7 ist ein chirurgisches Instrumentensystem 1 zur Verwendung bei einer Kniegelenkersatzoperation vorgesehen und weist einen intramedullären Stab 100, einen Referenzblock 200, wenigstens ein Lagerelement 300, einen Schnittblock 400 und wenigstens ein Kompensationselement 500 auf.

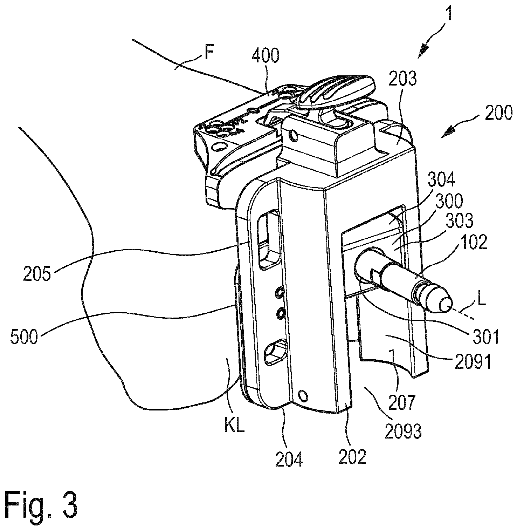

Der intramedulläre Stab 100 ist zwischen einem proximalen Stabende 101 und einem distalen Stabende 102 gerade längserstreckt und zum Einbringen in einen Markraum eines Femurs F eingerichtet (siehe Fig. 3). Der intramedulläre Stab 100 (nachfolgend abgekürzt: Stab) ist in eingesetztem Zustand koaxial zu einer proximodistalen Längsachse des Femurs F ausgerichtet. Im eingesetzten Zustand ragt das distale Stabende 102 stirnendseitig aus dem Femur F heraus und überragt dessen distale Kondylen, wobei anhand Fig. 3 lediglich eine laterale distale Kondyle KL des Femurs F ersichtlich ist.

Der Referenzblock 200 ist in axialer Richtung des Stabs 100 auf denselben aufschiebbar. Der Referenzblock 200 weist eine Blockrückseite 201, eine Blockvorderseite 202, eine Blockoberseite 203, eine Blockunterseite 204 sowie Blockaußenseiten 205, 206 auf. Die Blockrückseite 201 ist proximal orientiert. Das heißt eine nicht näher bezeichnete Flächennormale der Blockrückseite 201 ist entlang einer proximodistalen Achse (Fig. 1) ausgerichtet, wobei die Ausrichtung, wie nachfolgend noch näher erläutert wird, nicht notwendigerweise parallel sein muss. Die Blockvorderseite 202 liegt der Blockrückseite 201 distal gegenüber. Die Blockoberseite 203 ist anterior an dem Referenzblock 200 angeordnet. Die Blockunterseite 204 liegt der Blockoberseite 203 posterior gegenüber. Die beiden Blockaußenseiten 205, 206 liegen einander mediolateral gegenüber und können auch als laterale Blockaußenseite 205 und mediale Blockaußenseite 206 bezeichnet werden.

Die proximal orientierte Blockrückseite 201 ist zur Anlage an den besagten distalen Kondylen des Femurs F eingerichtet (siehe Fig. 3). Dabei ist der Referenzblock 200 mittels des Lagerelements 300 an dem distalen Stabende 102 gelagert.

Zu diesem Zweck weist das Lagerelement 300 eine Aufnahmebohrung 301 auf, welche zur koaxialen und radial formschlüssigen Aufnahme des distalen Stabendes 102 eingerichtet ist. Zudem ist das Lagerelement 300 relativ zu dem Referenzblock 200 wenigstens um eine anteroposterior orientierte Schwenkachse S schwenkbeweglich an dem Referenzblock 200 gelagert.

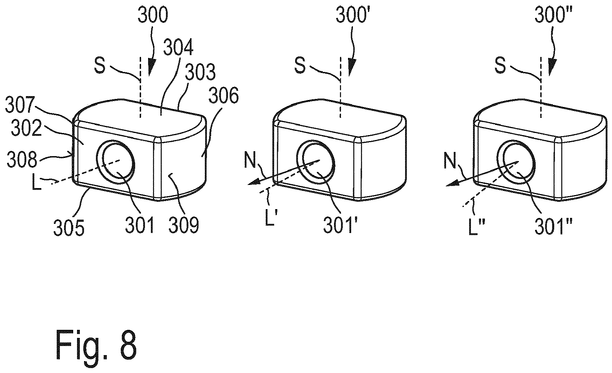

Das Lagerelement 300 weist eine proximal orientierte Rückseite 302, eine distal gegenüberliegende Vorderseite 303, eine anterior orientierte Oberseite 304, eine posterior gegenüberliegende Unterseite 305 sowie mediolateral gegenüberliegende Außenseiten 306, 307 auf. Letztere können auch als laterale Außenseite 306 und mediale Außenseite 307 bezeichnet werden. Die Aufnahmebohrung 301 ist entlang einer Längsachse L (siehe Fig. 8) zwischen der Rückseite 302 und der Vorderseite 303 längserstreckt. Die Längsachse L und die Schwenkachse S sind bei dem Lagerelement 300 orthogonal zueinander ausgerichtet. Wie nachfolgend noch näher beschrieben wird, sind auch hiervon abweichende Ausrichtungen vorgesehen.

Ein nicht näher bezeichneter Außenumfang des Stabs 100 und die Aufnahmebohrung 301 wirken in Radialrichtung der Aufnahmebohrung 301 im Wesentlichen spielfrei formschlüssig zusammen. In axialer Richtung der Aufnahmebohrung 301, d.h. entlang der Längsachse L und damit auch entlang der proximodistalen Achse, ist das Lagerelement 300 relativ zu dem Stab 100 gleitbeweglich. Zudem ist das Lagerelement 300 grundsätzlich in Umfangsrichtung des Stabs 100 um die Längsachse L rotierbar. Hiervon abgesehen ist keinerlei Relativbewegung zwischen dem Lagerelement 300 und dem Stab 100 möglich.

Durch die schwenkbewegliche Lagerung zwischen dem Referenzblock 200 und dem Lagerelement 300 kann der Referenzblock 200 um die Schwenkachse S relativ zu dem Stab 100 und damit auch dem Femur F in unterschiedlichen Winkelstellungen positioniert werden. Dies ermöglicht eine einfache und präzise Einstellung des Varus-Valgus-Winkels für die anschließend mittels des Schnittblocks 500 auszuführende distale Resektion des Femurs F.

Die Schwenkachse S schneidet die Längsachse L der Aufnahmebohrung 301 und damit auch die nicht gesondert bezeichnete Längsachse des Stabs 100.

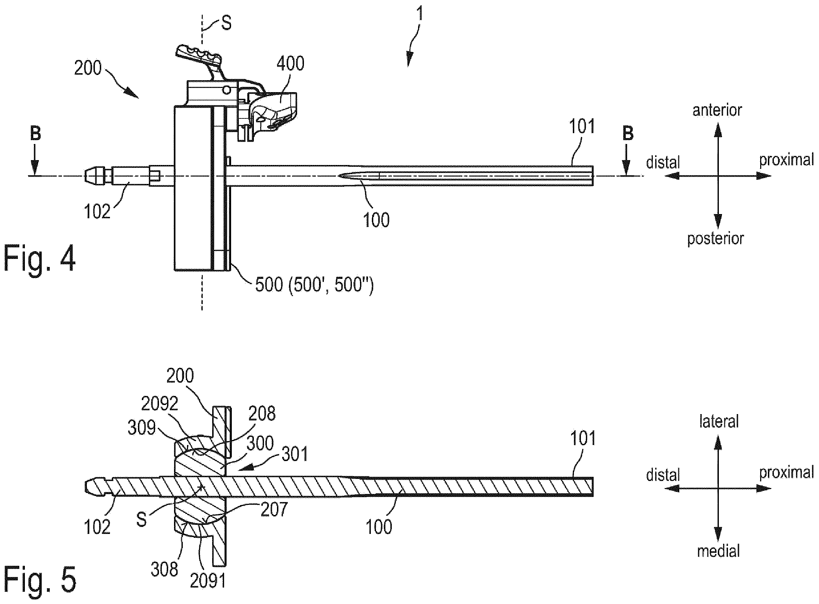

Bei der Ausführungsform nach den Fig. 1 bis 7 ist zwecks schwenkbeweglicher Lagerung eine Gleitführung mit Führungsflächen 207, 208 und Gleitflächen 308, 309 vorhanden (siehe insbesondere Fig. 5).

Vorliegend weist der Referenzblock 200 die Führungsflächen 207, 208 auf. Das Lagerelement weist dementgegen die Gleitflächen 308, 309 auf.

Die Führungsflächen 207, 208 liegen einander mediolateral gegenüber und sind jeweils - in Bezug auf eine anterior oder posterior gerichtete Blickrichtung (siehe Fig. 5) - kreisbogenförmig konkav gekrümmt. Die Gleitflächen 308, 309 sind komplementär zu den Führungsflächen 207, 208 geformt. Dementsprechend sind die Gleitflächen 308, 309 einander mediolateral gegenüberliegend an dem Lagerelement angeordnet sowie kreisbogenförmig konvex gekrümmt. Die Gleitflächen 308, 309 wirken mit den Führungsflächen 207, 208 um die Schwenkachse S rotatorisch gleitbeweglich zusammen. Proximodistal und damit entlang der Längsachse L wirken die besagten Flächen 207, 307, 208, 308 formschlüssig zusammen. Zudem liegt ein Formschluss in mediolateraler Richtung vor.

Die Führungsflächen 207, 208 können auch als mediale Führungsfläche 207 und laterale Führungsfläche 208 bezeichnet werden. Mit anderen Worten ausgedrückt liegt die mediale Führungsfläche 207 auf einer medialen Seite der Schwenkachse S und/oder des Stabs 100. Die laterale Führungsfläche 208 liegt dementgegen auf einer lateralen Seite der Schwenkachse und/oder des Stabs 100. Die Gleitflächen 308, 309 können auch als mediale Gleitfläche 308 und laterale Gleitfläche 309 bezeichnet werden. Die mediale Gleitfläche 308 ist an der medialen Außenseite 307 angeordnet oder durch diese selbst gebildet. Vorliegend ist letzteres der Fall. Die laterale Gleitfläche 309 ist durch die laterale Außenseite 306 gebildet.

Vorliegend sind die Führungsflächen 207, 208 jeweils parallel zur Schwenkachse S gerade längserstreckt. Infolge der geraden und parallelen Längserstreckung bilden die Führungsflächen 207, 208 gleichsam eine entlang der Schwenkachse S erstreckte Führungsbahn E aus (siehe insbesondere Fig. 2). Der Referenzblock 200 und das Lagerelement 300 sind relativ zueinander translatorisch entlang der Führungsbahn E verlagerbar. Hierdurch kann der Referenzblock 200 relativ zu dem Stab 100 und damit auch zu dem Femur F in unterschiedlichen Positionen in Bezug auf die anteroposteriore Achse positioniert werden. Mit anderen Worten kann der Referenzblock 200 in Bezug auf seine nicht näher bezeichnete Hochachse unterschiedlich an dem distalen Femur F positioniert werden. Hierdurch kann die Position des Referenzblocks 200 einfach und präzise an unterschiedlich große Femurknochen angepasst werden. Wie anhand Fig. 3 gezeigt ist, ist der Referenzblock 200 entlang der Führungsbahn E (Fig. 2) relativ zu dem Lagerelement 300 zwischen einer unteren Endlage und einer oberen Endlage verschieblich. In bspw. Fig. 3 ist eine Positionierung im Bereich der unteren Endlage gezeigt. Die obere Endlage wird eingenommen, wenn der Referenzblock 200 ausgehend von der in Fig. 3 gezeigten Position in Bezug auf die Zeichenebene nach oben und bis zum Erreichen eines unteren Endes der Führungsflächen 207, 208 verlagert wird.

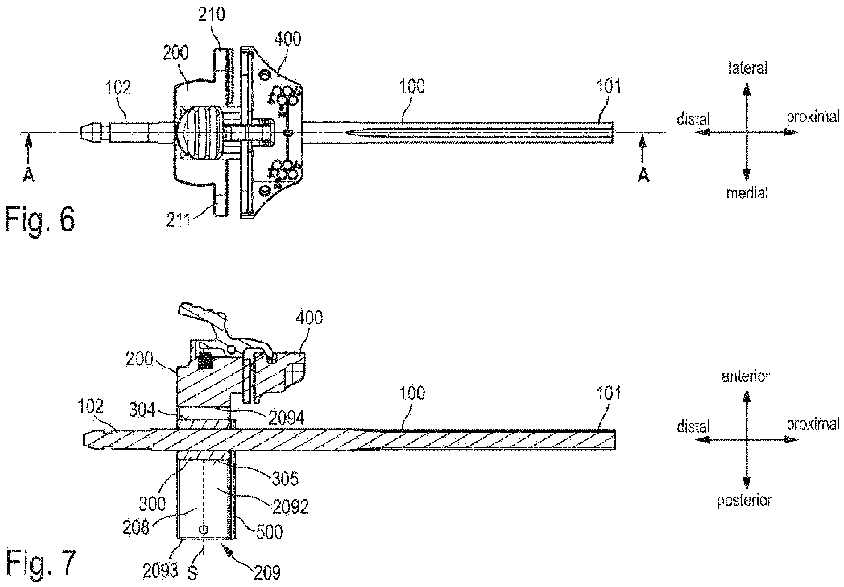

Vorliegend weist der Referenzblock 200 einen von der Blockrückseite 201 auf die distal gegenüberliegende Blockvorderseite 202 reichenden Aufnahmeschlitz 209 auf. Der Aufnahmeschlitz 209 ist parallel zu der Schwenkachse S und/oder der Führungsbahn E gerade längserstreckt und weist mediolateral gegenüberliegende Innenwandungen 2091, 2092 auf, an welchen die Führungsflächen 207, 208 angeordnet und/oder ausgebildet sind. Vorliegend ist letzteres der Fall. Zudem weist der Aufnahmeschlitz eine vorliegend an der Blockunterseite 204 angeordnete Einführöffnung 2093 auf. Die Einführöffnung 2093 ist zum Einführen des Lagerelements 300 in den Aufnahmeschlitz 209 eingerichtet. Der Aufnahmeschlitz 209 weist zudem eine Stirnwand 2094 auf. Die Stirnwand 2094 liegt der Einführöffnung 2093 entlang der Schwenkachse S anterior gegenüber. Die Stirnwand 2094 bildet einen Anschlag für das Lagerelement 300, genauer: dessen Oberseite 304 (siehe Fig. 7).

Bei der gezeigten Ausführungsform unterteilt der Aufnahmeschlitz 209 den Referenzblock 200 in einen lateralen Blockabschnitt 210 und einen medialen Blockabschnitt 211 (siehe insbesondere Fig. 6). Dementsprechend ist die Blockrückseite 201 in eine laterale Blockrückseite und eine mediale Blockrückseite untergliedert (jeweils ohne Bezugszeichen).

Wie anhand Fig. 8 gezeigt ist, weist das chirurgische Instrumentensystem 1 vorliegend mehrere unterschiedliche Lagerelemente 300, 300', 300" auf. Diese können auch als erstes Lagerelement 300, zweites Lagerelement 300' und drittes Lagerelement 300" bezeichnet werden. Die Lagerelemente 300, 300', 300" weisen eine im Wesentlichen identische Gestaltung und Funktionsweise auf und unterscheiden sich lediglich hinsichtlich der Ausrichtung der Längsachse L, L', L" der jeweiligen Aufnahmebohrung 301, 301', 301". Die Längsachsen L, L', L" sind jeweils unterschiedlich geneigt längserstreckt. Die unterschiedlichen Neigungswinkel sind in Bezug auf eine Normalenrichtung N der Blockrückseite 201 verdeutlicht. Die Normalenrichtung N ist in Fig. 8 exemplarisch bei dem zweiten Lagerelement 300' und dem dritten Lagerelement 300" eingezeichnet. Bei dem ersten Lagerelement 300 fällt die Normalenrichtung N mit der Längsachse L zusammen. Die Lagerelemente 300, 300', 300" sind jeweils gegeneinander austauschbar an dem Referenzblock 200 anbringbar.

Zwecks Demontage kann das Lagerelement 300 - ausgehend von der anhand Fig. 2 gezeigten Konfiguration - in posteriorer Richtung aus dem Aufnahmeschlitz 209 herausgezogen werden. Die Montage erfolgt kinematisch umgekehrt. Durch die unterschiedlich geneigten Längsachsen L, L', L" kann der Referenzblock 200 wahlweise in unterschiedlichen Neigungswinkeln in Bezug auf den Femur F positioniert werden. Im Speziellen kann hierdurch der sogenannte Flexion-Extensions-Winkel einfach und präzise angepasst werden. Die Längsachsen L, L', L" sind in festen Winkelschritten voneinander unterschiedlich. Folglich kann die Anpassung der Winkelausrichtung des Referenzblocks 200 in festgelegten, definierten Schritten erfolgen. Die Einstellung der Neigung des Referenzblocks 200 erfolgt hierbei um eine mediolateral ausgerichtete Achse, die sowohl die Schwenkachse S als auch die Längsachse L schneidet.

Bei der gezeigten Ausführungsform weist das chirurgische Instrumentensystem 1 zudem den besagten Schnittblock 400 auf. Die Funktion und Gestaltung des Schnittblocks 400 ist dem Fachmann grundsätzlich bekannt. Der Schnittblock 400 dient der Aufnahme und Führung eines Sägeblatts zur Resektion der distalen Kondylen des Femurs F. Der Schnittblock 400 ist mittels einer Fixiervorrichtung 212 lösbar an dem Referenzblock 200 angebracht. Details der Fixiervorrichtung 212 und des Schnittblocks 400 sind hinsichtlich der vorliegenden Erfindung nicht von wesentlicher Bedeutung. Auf weitere diesbezügliche Erläuterungen kann deshalb verzichtet werden.

Das Kompensationselement 500 ist zur Kompensation einer nicht näher dargestellten Abnutzung der lateralen distalen Kondyle KL eingerichtet und zu diesem Zweck lösbar an der Blockrückseite 201 festgelegt. Das Kompensationselement weist eine nicht näher bezeichnete proximodistale Dicke auf, die in etwa der besagten Abnutzung der lateralen Kondyle KL entspricht. Zur Kompensation unterschiedlich stark ausgeprägter Abnutzungen weist das chirurgische Instrumentensystem 1 vorliegend mehrere unterschiedliche Kompensationselemente 500, 500', 500" auf (siehe Fig. 4). Dabei ist allein aus zeichnerischen Gründen lediglich das Kompensationselement 500 im Detail dargestellt. Die Kompensationselemente 500, 500', 500" weisen unterschiedliche proximodistale Dicken auf und sind gegeneinander austauschbar an der Blockrückseite 201 anbringbar. Die Kompensationselemente 500, 500', 500" sowie deren Anbringung an der Blockrückseite 201 sind hinsichtlich der vorliegenden Erfindung nicht wesentlich. Auf weitere diesbezügliche Erörterungen kann deshalb verzichtet werden.

Der intramedulläre Stab 100 weist bei der gezeigten Ausführungsform eine Länge von 175 mm auf. Bei zeichnerisch nicht dargestellten Ausführungsformen beträgt die Länge zwischen 120 mm und 250 mm.

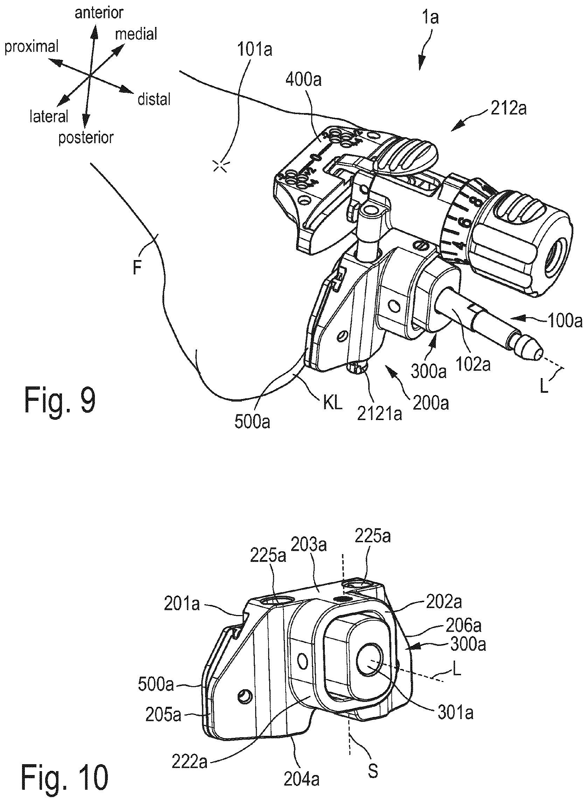

Die Fig. 9 bis 14 zeigen eine weitere Ausführungsform eines erfindungsgemäßen chirurgischen Instrumentensystems 1a. Dessen Funktion und Gestaltung sind im Wesentlichen identisch mit dem chirurgischen Instrumentensystem 1 nach den Fig. 1 bis 7. Zur Vermeidung von Wiederholungen wird nachfolgend in erster Linie auf wesentliche Unterschiede des chirurgischen Instrumentensystems 1a gegenüber dem chirurgischen Instrumentensystem 1 nach den Fig. 1 bis 7 eingegangen. Funktionsgleiche Bauteile und/oder Abschnitte sind mit identischen Bezugszeichenziffern unter Hinzufügung des Kleinbuchstabens "a" gekennzeichnet. Funktionsgleiche Bauteile und/oder Abschnitte werden nicht gesondert erläutert. Stattdessen wird ausdrücklich auf das zu dem chirurgischen Instrumentensystem 1 Gesagte Bezug genommen und verwiesen.

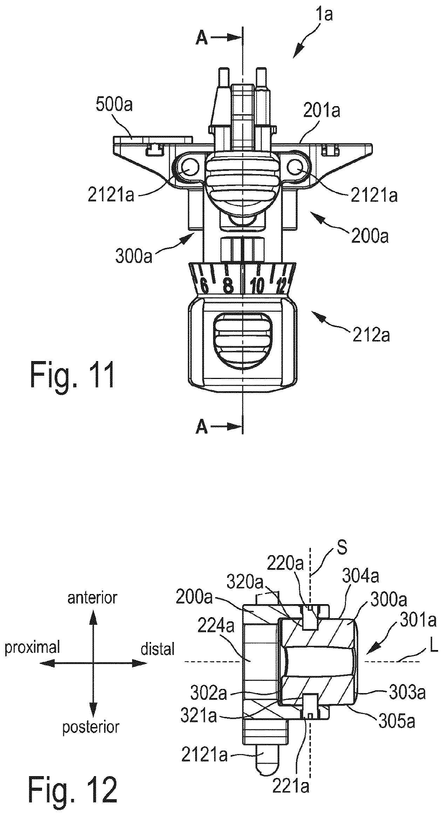

Das chirurgische Instrumentensystem 1a unterscheidet sich in erster Linie durch eine unterschiedliche Umsetzung der Schwenkbeweglichkeit zwischen dem Referenzblock 200a und dem Lagerelement 300a. Zwecks schwenkbeweglicher Lagerung ist vorliegend eine Achsführung vorgesehen. Die Achsführung weist koaxial zu der Schwenkachse S orientierte Achselemente 220a, 221a sowie entsprechende Achsaufnahmen 320a, 321a auf (siehe insbesondere Fig. 12). Vorliegend sind die Achselemente 220a, 221a dem Referenzblock 200a zugeordnet. Die Achsaufnahmen 320a, 321a sind an dem Lagerelement 300a angeordnet und/oder ausgebildet.

Die Achselemente 220a, 221a sind koaxial zueinander ausgerichtet. Entsprechendes gilt sinngemäß für die Achsaufnahmen 320a, 321a. Die Achselemente 220a, 221a und die Achsaufnahmen 320a, 321a wirken um die Schwenkachse S gleitbeweglich zusammen. Hierfür greift jeweils eines der Achselemente 220a, 221a axial in eine der Achsaufnahmen 320a, 321a ein. Sowohl die Achselemente 220a, 221a als auch die Achsaufnahmen 320a, 321a sind vorliegend orthogonal zu der Längsachse der Aufnahmebohrung 301a ausgerichtet.

In Bezug auf die Längsachse L kann vorliegend auch von einem anterioren Achselement 220a und einem posterioren Achselement 221a gesprochen werden. Dies gilt mutatis mutandis auch für die Achsaufnahmen 320a, 321a.

Die anteriore Achsaufnahme 320a ist posterior in eine Oberseite 304a des Lagerelements 300a eingesenkt. Die posteriore Achsaufnahme 321a ist anterior in eine Unterseite 305a des Lagerelements 301a eingesenkt. Beide Achsaufnahmen 320a, 321a sind jeweils in Form einer Sackbohrung in das Lagerelement 300a eingebracht.

Die Achselemente 220a, 221a sind jeweils lösbar an dem Referenzblock 200a festgelegt und vorliegend jeweils in Form einer Senkschraube mit einem hierfür geeigneten Gewinde des Referenzblocks 200a verschraubt. Zum Lösen der Achselemente 220a, 221a sind diese jeweils mit einer nicht näher bezeichneten Werkzeugfläche zur Aufnahme eines komplementären Werkzeugs ausgestattet.

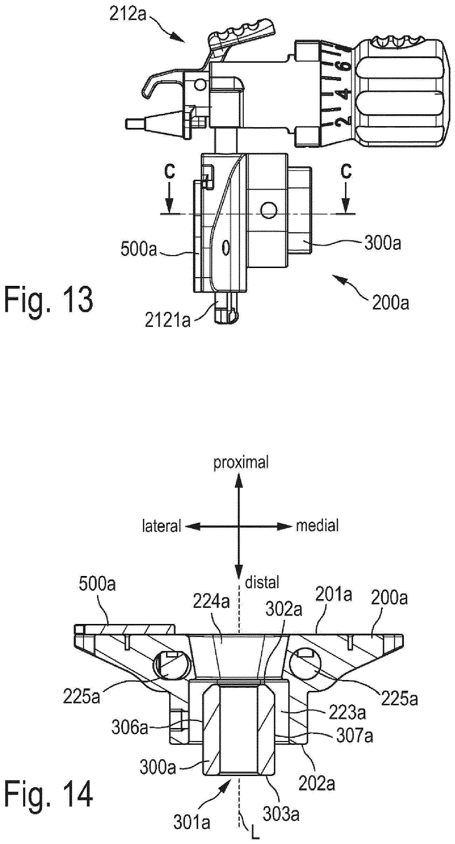

Das Lagerelement 300a ist bei der gezeigten Ausführungsform in einem hohlzylindrischen Vorsprung 222a des Referenzblocks 200a gelagert. Der Vorsprung 222a weist eine Aufnahmeaussparung 223a auf (Fig. 14), in welcher das Lagerelement 300a auf die vorbeschriebene Weise schwenkbeweglich gelagert ist. Die Aufnahmeaussparung 223a mündet in proximaler Richtung in einen Durchlass 224a (Fig. 14). Der Durchlass 224a dient der Aufnahme des distalen Stabendes 102a, das ausgehend von der Blockrückseite 201a durch den Durchlass 224a und von dort weiter über die Aufnahmebohrung 301a durch den Referenzblock 200a geführt werden kann.

Im Unterschied zu dem Lagerelement 300 des chirurgischen Instrumentensystems 1 ist das Lagerelement 300a nicht anteroposterior entlang der Schwenkachse S linear verschieblich an dem Referenzblock 200a gelagert.

Um dennoch eine anteroposteriore Einstellbarkeit des Schnittblocks 400a zu ermöglichen, ist die Fixiervorrichtung 212a relativ zu dem Referenzblock 200a entsprechend verlagerbar. Zu diesem Zweck weist der Referenzblock 200a Führungsbohrungen 225a auf (Fig. 10), in welche Führungszapfen 2121a der Fixiervorrichtung 212a axial eingreifen.

Im Übrigen sind die weitere Funktion und Gestaltung der Fixiervorrichtung 212a hinsichtlich der vorliegenden Erfindung nicht wesentlich, so dass weitere diesbezügliche Erläuterungen entbehrlich sind.

In Bezug auf die Funktion des Kompensationselements 500a gilt das zu dem chirurgischen Instrumentensystem 1 Gesagte sinngemäß. Zudem können wiederum mehrere unterschiedliche Kompensationselemente mit unterschiedlicher proximodistaler Dicke vorhanden sein.

Unter Bezugnahme auf Fig. 8 kann das chirurgische Instrumentensystem 1a wiederum mehrere unterschiedliche Lagerelemente mit unterschiedlich geneigten Längsachsen aufweisen, die gegeneinander austauschbar an dem Referenzblock 200a anbringbar sind. Zum Austausch der Lagerelemente können die Achselemente von dem Referenzblock 200a gelöst und erneut befestigt werden. Dies mittels der vorbeschriebenen Schraubverbindung.

## Patentansprüche

1. Chirurgisches Instrumentensystem (1, 1a) zur Verwendung bei einer Kniegelenkersatzoperation, aufweisend
einen intramedullären Stab (100, 100a), welcher zwischen einem proximalen Stabende (101, 101a) und einem distalen Stabende (102, 102a) längserstreckt und zum Einbringen in den Markraum eines Femurs (F) eingerichtet ist, und
einen auf das distale Stabende (102, 102a) aufgeschobenen oder aufschiebbaren Referenzblock (200, 200a) mit einer proximal orientierten Blockrückseite (201, 201a), welche zur Anlage an distalen Kondylen des Femurs (F) eingerichtet ist,
wobei der Referenzblock (200, 200a) mittels eines Lagerelements (300, 300a) an dem distalen Stabende (102, 102a) gelagert oder lagerbar ist, wobei das Lagerelement (300, 300a) eine Aufnahmebohrung (301, 301a) zur koaxialen und radial formschlüssigen Aufnahme des distalen Stabendes (102, 102a) aufweist und relativ zu dem Referenzblock (200, 200a) wenigstens um eine anteroposterior orientierte Schwenkachse (S) schwenkbeweglich an demselben gelagert ist,
**dadurch gekennzeichnet, dass** das wenigstens eine Lagerelement (300, 300a) lösbar an dem Referenzblock (200, 200a) gelagert ist, und dass mehrere unterschiedliche Lagerelemente (300, 300', 300") mit unterschiedlich geneigt längserstreckten Aufnahmebohrungen (301, 301', 301") vorhanden sind,
wobei die Aufnahmebohrungen (301, 301', 301") in Bezug auf eine Normalenrichtung der Blockrückseite (201, 201a) unterschiedlich in anteriorer und/oder posteriorer Richtung geneigt und zur Einstellung eines Flexions-Extensions-Winkels des Referenzblocks (200, 200a) an demselben gegeneinander austauschbar lagerbar sind.

2. Chirurgisches Instrumentensystem (1) nach Anspruch 1, **dadurch gekennzeichnet, dass** der Referenzblock (200) eine Gleitführung mit mediolateral gegenüberliegenden kreisbogenförmig konkav gekrümmten Führungsflächen (207, 208) aufweist, und dass das Lagerelement (300) mediolateral gegenüberliegende kreisbogenförmig konvex gekrümmte Gleitflächen (308, 309) aufweist, wobei die Gleitflächen (308, 309) um die Schwenkachse (S) rotatorisch gleitbeweglich und proximodistal sowie mediolateral translatorisch formschlüssig mit den Führungsflächen (207, 208) zusammenwirken.

3. Chirurgisches Instrumentensystem (1) nach Anspruch 2, **dadurch gekennzeichnet, dass** die Führungsflächen (207, 208) jeweils parallel zu der Schwenkachse (S) gerade längserstreckt sind, wobei die Gleitflächen (308, 309) entlang der Schwenkachse (S) translatorisch gleitbeweglich mit den Führungsflächen (207, 208) zusammenwirken.

4. Chirurgisches Instrumentensystem (1) nach Anspruch 2 oder 3, **dadurch gekennzeichnet, dass** der Referenzblock (200) einen von der Blockrückseite (201) auf eine distal gegenüberliegende Blockvorderseite (202) reichenden sowie parallel zu der Schwenkachse (S) gerade längserstreckten Aufnahmeschlitz (209) mit mediolateral gegenüberliegenden Innenwandungen (2091, 2092) aufweist, an welchen die Führungsflächen (207, 208) angeordnet und/oder ausgebildet sind, wobei der Aufnahmeschlitz (209) zum Einführen des Lagerelements (300) eine anterior an einer Blockoberseite (203) oder posterior an einer Blockunterseite (204) des Referenzblocks (200) angeordnete Einführöffnung (2093) aufweist.

5. Chirurgisches Instrumentensystem (1a) nach Anspruch 1, **dadurch gekennzeichnet, dass** der Referenzblock (200a) eine Achsführung mit wenigstens einem koaxial zu der Schwenkachse (S) orientierten Achselement (220a, 221a) aufweist, und dass das Lagerelement (300a) wenigstens eine koaxial zu der Schwenkachse (S) orientierte Achsaufnahme (320a, 321a) aufweist, wobei das Achselement (220a, 221a) um die Schwenkachse (S) gleitbeweglich in die Achsaufnahme (320a, 321a) eingreift.

6. Chirurgisches Instrumentensystem (1, 1a) nach einem der vorhergehenden Ansprüche, **dadurch gekennzeichnet, dass** mehrere unterschiedliche Kompensationselemente (500, 500a, 500', 500") mit unterschiedlichen proximodistalen Dicken vorhanden und zur Kompensation unterschiedlich stark ausgeprägter Abnutzungen der distalen Kondylen an der Blockrückseite (201, 201a) gegeneinander austauschbar anbringbar sind.

7. Chirurgisches Instrumentensystem (1, 1a) nach einem der vorhergehenden Ansprüche, **dadurch gekennzeichnet, dass** ein Schnittblock (400, 400a) vorhanden ist, welcher zu einer Schnittführung an den distalen Kondylen des Femurs (F) eingerichtet und an einer Fixiervorrichtung (212, 212a) des Referenzblocks (200, 200a) lösbar fixiert oder fixierbar ist.

8. Chirurgisches Instrumentensystem (1, 1a) nach einem der vorhergehenden Ansprüche, **dadurch gekennzeichnet, dass** der intramedulläre Stab (100, 100a) eine Länge zwischen 120 mm und 250 mm, bevorzugt zwischen 150 mm und 220 mm, besonders bevorzugt zwischen 170 mm und 200 mm, aufweist.

## Claims

1. Surgical instrument system (1, 1a) for use in a knee joint replacement operation, having
an intramedullary rod (100, 100a) which extends lengthwise between a proximal rod end (101, 101a) and a distal rod end (102, 102a) and is configured for introduction into the medullary space of a femur (F), and a reference block (200, 200a) which is pushed or can be pushed onto the distal rod end (102, 102a) and has a proximally oriented block rear side (201, 201a) configured to bear on distal condyles of the femur (F),
wherein the reference block (200, 200a) is mounted or mountable on the distal rod end (102, 102a) by means of a bearing element (300, 300a), wherein the bearing element (300, 300a) has a reception bore (301, 301a) for coaxial and radially form-fit reception of the distal rod end (102, 102a) and is mounted on the reference block (200, 200a) in such a way as to be pivotable relative thereto at least about a pivot axis (S) oriented in an anteroposterior direction,
**characterized in that** the at least one bearing element (300, 300a) is mounted releasably on the reference block (200, 200a), and **in that** a plurality of different bearing elements (300, 300', 300'') are provided that have differently inclined elongate reception bores (301, 301', 301''),
wherein the reception bores (301, 301', 301'') are inclined differently, relative to a normal direction of the block rear side (201, 201a), in an anterior and/or posterior direction and can be mounted interchangeably on the reference block (200, 200a) for setting a flexion-extension angle of the latter.

2. Surgical instrument system (1) according to Claim 1, **characterized in that** the reference block (200) has a slide guide with guide faces (207, 208) that are concavely curved in the shape of an arc of a circle and lie opposite each other in a mediolateral direction, and **in that** the bearing element (300) has slide faces (308, 309) that are convexly curved in the shape of an arc of a circle and lie opposite each other in a mediolateral direction, wherein the slide faces (308, 309) are able to slide in rotation about the pivot axis (S) and formfittingly cooperate with the guide faces (207, 208) in translation in a proximodistal and mediolateral direction.

3. Surgical instrument system (1) according to Claim 2, **characterized in that** the guide faces (207, 208) each extend lengthwise in a straight line parallel to the pivot axis (S), wherein the slide faces (308, 309) cooperate with the guide faces (207, 208) along the pivot axis (S) in a translational sliding movement.

4. Surgical instrument system (1) according to Claim 2 or 3, **characterized in that** the reference block (200) has a reception slot (209), which reaches from the block rear side (201) to a block front side (202) lying opposite in a distal direction, extends lengthwise in a straight line parallel to the pivot axis (S) and has inner walls (2091, 2092), which lie opposite each other in a mediolateral direction and on which the guide faces (207, 208) are arranged and/or formed, wherein the reception slot (209) has, for the insertion of the bearing element (300), an insertion opening (2093) arranged in an anterior direction on a block upper side (203) or in a posterior direction on a block lower side (204) of the reference block (200).

5. Surgical instrument system (1a) according to Claim 1, **characterized in that** the reference block (200a) has an axial guide with at least one axial element (220a, 221a) oriented coaxially with respect to the pivot axis (S), and **in that** the bearing element (300a) has at least one axial recess (320a, 321a) oriented coaxially with respect to the pivot axis (S), wherein the axial element (220a, 221a) engages in the axial recess (320a, 321a) in such a way as to be able to slide about the pivot axis (S).

6. Surgical instrument system (1, 1a) according to any one of the preceding claims, **characterized in that** a plurality of different compensation elements (500, 500a, 500', 500'') having different proximodistal thicknesses are provided, which can be applied mutually interchangeably on the block rear side (201, 201a) in order to compensate for different degrees of wear of the distal condyles.

7. Surgical instrument system (1, 1a) according to any one of the preceding claims, **characterized in that** a cutting block (400, 400a) is provided, which is configured for guiding the cutting on the distal condyles of the femur (F) and is releasably fixed or fixable on a fixing device (212, 212a) of the reference block (200, 200a).

8. Surgical instrument system (1, 1a) according to any one of the preceding claims, **characterized in that** the intramedullary rod (100, 100a) has a length of between 120 mm and 250 mm, preferably of between 150 mm and 220 mm, particularly preferably of between 170 mm and 200 mm.

## Revendications

1. Système d'instrument chirurgical (1, 1a) destiné à être utilisé dans une opération de remplacement de l'articulation du genou, présentant
une tige intramédullaire (100, 100a) qui s'étend longitudinalement entre une extrémité de tige proximale (101, 101a) et une extrémité de tige distale (102, 102a) et qui est adaptée pour être insérée dans le canal médullaire d'un fémur (F), et
un bloc de référence (200, 200a) enfilé ou pouvant être enfilé sur l'extrémité de tige distale (102, 102a), avec un côté arrière de bloc (201, 201a) orienté de manière proximale, qui est adapté pour s'appuyer sur des condyles distaux du fémur (F),
le bloc de référence (200, 200a) étant logé ou pouvant être logé au moyen d'un élément de palier (300, 300a) sur l'extrémité de tige distale (102, 102a), l'élément de palier (300, 300a) présentant un alésage de réception (301, 301a) pour la réception coaxiale et radiale par complémentarité de forme de l'extrémité de tige distale (102, 102a) et étant logé sur le bloc de référence (200, 200a) de manière à pouvoir se déplacer en pivotement par rapport à celui-ci au moins autour d'un axe de pivotement (S) orienté de manière antéropostérieure,
**caractérisé en ce que** l'au moins un élément de palier (300, 300a) est logé de manière amovible sur le bloc de référence (200, 200a), et **en ce que** plusieurs éléments de palier différents (300, 300', 300'') sont présents avec des alésages de réception (301, 301', 301'') s'étendant longitudinalement avec des inclinaisons différentes,
les alésages de réception (301, 301', 301") étant inclinés différemment dans la direction antérieure et/ou postérieure par rapport à une direction normale du côté arrière du bloc (201, 201a) et pouvant être logés de manière interchangeable sur le bloc de référence (200, 200a) pour régler un angle de flexion-extension de celui-ci.

2. Système d'instrument chirurgical (1) selon la revendication 1, **caractérisé en ce que** le bloc de référence (200) présente un guide coulissant avec des surfaces de guidage (207, 208) opposées médiolatéralement, courbées de manière concave en forme d'arc de cercle, et **en ce que** l'élément de palier (300) présente des surfaces de coulissement (308, 309) opposées médiolatéralement, courbées de manière convexe en forme d'arc de cercle, les surfaces de coulissement (308, 309) coopérant avec les surfaces de guidage (207, 208) de manière à pouvoir se déplacer en coulissement par rotation autour de l'axe de pivotement (S) ainsi qu'en translation de manière proximodistale et médiolatérale par complémentarité de forme.

3. Système d'instrument chirurgical (1) selon la revendication 2, **caractérisé en ce que** les surfaces de guidage (207, 208) sont chacune étendues longitudinalement en ligne droite parallèlement à l'axe de pivotement (S), les surfaces de coulissement (308, 309) coopérant avec les surfaces de guidage (207, 208) de manière à pouvoir se déplacer en coulissement par translation le long de l'axe de pivotement (S).

4. Système d'instrument chirurgical (1) selon la revendication 2 ou 3, **caractérisé en ce que** le bloc de référence (200) présente une fente de réception (209) s'étendant depuis le côté arrière du bloc (201) jusqu'à un côté avant du bloc (202) distalement opposée et s'étendant longitudinalement en ligne droite parallèlement à l'axe de pivotement (S), avec des parois intérieures (2091, 2092) opposées médiolatéralement, sur lesquelles les surfaces de guidage (207, 208) sont agencées et/ou réalisées, la fente de réception (209) présentant, pour l'introduction de l'élément de palier (300), une ouverture d'introduction (2093) agencée antérieurement sur un côté supérieur de bloc (203) ou postérieurement sur un côté inférieur de bloc (204) du bloc de référence (200).

5. Système d'instrument chirurgical (1a) selon la revendication 1, **caractérisé en ce que** le bloc de référence (200a) présente un guide d'axe avec au moins un élément d'axe (220a, 221a) orienté coaxialement à l'axe de pivotement (S), et **en ce que** l'élément de palier (300a) présente au moins un logement d'axe (320a, 321a) orienté coaxialement à l'axe de pivotement (S), l'élément d'axe (220a, 221a) s'engageant dans le logement d'axe (320a, 321a) de manière à pouvoir se déplacer en coulissement autour de l'axe de pivotement (S).

6. Système d'instrument chirurgical (1, 1a) selon l'une quelconque des revendications précédentes, **caractérisé en ce que** plusieurs éléments de compensation différents (500, 500a, 500', 500'') ayant des épaisseurs proximodistales différentes sont présents et peuvent être montés de manière interchangeable sur le côté arrière du bloc (201, 201a) pour compenser des usures des condyles distaux d'intensité différente.

7. Système d'instrument chirurgical (1, 1a) selon l'une quelconque des revendications précédentes, **caractérisé en ce qu'**un bloc de coupe (400, 400a) est présent, qui est adapté pour effectuer une coupe au niveau des condyles distaux du fémur (F) et qui est fixé ou peut être fixé de manière amovible à un dispositif de fixation (212, 212a) du bloc de référence (200, 200a).

8. Système d'instrument chirurgical (1, 1a) selon l'une quelconque des revendications précédentes, **caractérisé en ce que** la tige intramédullaire (100, 100a) présente une longueur comprise entre 120 mm et 250 mm, de préférence entre 150 mm et 220 mm, de manière particulièrement préférée entre 170 mm et 200 mm.
